# EUROPEAN PATENT SPECIFICATION

(11) **EP 4 262 569 B1**
(45) Date of publication and mention of the grant of the patent: **16.09.2026**
(21) Application number: 21844895.9
(22) Date of filing: 21.12.2021
(51) Int. Cl.: A61B 8/08, A61M 25/01

(54) **ECHOGENIC MULTILAYER MEDICAL DEVICE**
ECHOGENE MEHRSCHICHTIGE MEDIZINISCHE VORRICHTUNG
DISPOSITIF MÉDICAL MULTICOUCHE ÉCHOGÈNE

(30) Priority: 21.12.2020 US 202063128504 P; 20.12.2021 US 202117645218
(43) Date of publication of application: 25.10.2023
(73) Proprietor: Medtronic, Inc., Minneapolis, Minnesota 55432 (US)
(72) Inventor: SCHMIDT, Elliot C., Mounds View, Minnesota 55112 (US); PISCHLAR, Jesse J., Mounds View, Minnesota 55112 (US)
(74) Representative: Zimmermann & Partner Patentanwälte mbB
(86) International application number: PCT/US2021/064672
(87) International publication number: WO 2022/140430

(56) References cited:
- WO-A1-2021/003342
- CN-U- 211 884 905
- US-A1- 2009 131 910
- US-A1- 2012 059 308
- US-A1- 2013 053 770

## Description

### TECHNICAL FIELD

This disclosure relates to medical devices including elongated members introducible into a body of a patient.

### BACKGROUND

Medical devices may be advanced into the vasculature of a patient to introduce an implantable medical device (IMD), or therapeutic agent to a treatment site. IMDs may be configured for delivery into a selected location within a patient, such as different chambers of a patient's heart, using such catheters. Medical imaging devices and techniques, such as fluoroscopy, may be used to aid in the positioning of medical devices within the patient for the delivery of an IMD,
US 2013/053770 A1 relates to a medical balloon and a balloon catheter assembly.
US 2009/131910 A1 relates to visualization of a catheter viewed under ultrasound imaging. US 2012/059308 A1 relates to a peripheral nerve block catheter.
CN 211884905 U relates to a balloon dilatation catheter and balloon thereof.

### SUMMARY

The present invention is directed to a medical device as defined in claim 1. Embodiments of the invention are recited in the dependent claims.

The use of fluoroscopy imaging techniques to aid in the positioning of medical devices includes several drawbacks, such as radiation exposure to patients and clinicians, clinician personal protective equipment requirements, such as lead vests or aprons, lack of soft tissue visibility using x-rays, and the need for large, expensive fluoroscopy or other medical imaging equipment. The disclosed medical device includes echogenic regions that enable a clinician to use other imaging devices and techniques, such as ultrasound, to aid in the positioning of medical devices at a selected location within a patient for the delivery of IMD or therapeutic agent. As one example, a medical device may include at least one echogenic region near a distal end of the medical device. The at least one echogenic region may be used to determine a position of the distal end of the medical device relative to a selected location, such as a particular region of the heart of the patient. Once positioned at the selected location, an IMD, such as, for example, a medical electrical lead, may be advanced through a lumen of the medical device to the selected location.

According to the claimed invention, a medical device includes a first layer defining an elongate body extending along a longitudinal axis from a proximal end to a distal end and defining a lumen extending longitudinally within the elongate body. The medical device further includes a second layer disposed on and radially adjacent to the first layer. At least a portion of the second layer defines an echogenic region. The echogenic region includes an echogenic metallic or ceramic material dispersed in the second layer. The echogenic region is configured to diffusely scatter a soundwave.

**In** some examples, a kit may include a first medical device and a second medical device. The first medical device may include a first layer defining an elongate body extending along a longitudinal axis from a proximal end to a distal end and defining a lumen extending longitudinally within the elongate body. The medical device may further include a second layer disposed on and radially adjacent to the first layer. At least a portion of the second layer defines an echogenic region. The echogenic region may include an echogenic metallic or ceramic material dispersed in the second layer. The echogenic region may be configured to diffusely scatter a soundwave. The second medical device may be sized for delivery out of the distal end of the elongate body and configured for at least one of therapy delivery or sensing.

**In** some examples, a method may include advancing a first medical device toward a selected location within a patient. The first medical device may include a first layer defining an elongate body extending along a longitudinal axis from a proximal end to a distal end and defining a lumen extending longitudinally within the elongate body. The first layer may include a first polymer. The medical device may further include a second layer disposed on and radially adjacent to the first layer. The second layer may include a second polymer. At least a portion of the second layer defines an echogenic region. The echogenic region may include the second polymer, and an echogenic metallic or ceramic material dispersed in the second polymer. The echogenic region may be configured to diffusely scatter a soundwave. The method also may include identifying at least one of a position, an orientation, or a trajectory of the distal portion of the first medical device relative to the selected location based on a soundwave reflected by the echogenic member. The method also may include advancing a second medical device through the lumen and out the distal end of the elongate body to the selected location for at least one of therapy delivery or sensing.

In some examples, a method of assembling a medical device may include forming a first layer defining an elongate body extending along a longitudinal axis from a proximal end to a distal end and defining a lumen extending longitudinally within the elongate body. The first layer may include a first polymer. The method also may include forming a second layer disposed on and radially adjacent to the first layer. The second layer may include a second polymer. At least a portion of the second layer defines an echogenic region. The echogenic region may include the second polymer, and an echogenic metallic or ceramic material dispersed in the second polymer. The echogenic region may be configured to diffusely scatter a soundwave.

The details of one or more examples are set forth in the accompanying drawings and the description below. Other features, objects, and advantages will be apparent from the description and drawings, and from the claims.

### BRIEF DESCRIPTION OF THE DRAWINGS

FIG. 1A is a conceptual side view of an example medical device having an echogenic region.
FIG. 1B is a conceptual side view of the medical device illustrated in FIG. 1A in an expanded configuration.
FIG. 2 is a conceptual diagram illustrating an example medical device having two echogenic regions and a handle assembly.
FIG. 3 is a conceptual diagram illustrating a right side of a heart in which a distal portion of an example medical device having an echogenic region is disposed.
FIG. 4 is a flow diagram illustrating an example method for manufacturing an example medical device.
FIG. 5 is a flow diagram illustrating an example method of delivering an example IMD to a selected location using an example medical device.

### DETAILED DESCRIPTION

The disclosure describes example systems, devices, and techniques for positioning echogenic medical devices at a selected location within a patient to deliver another medical device or therapeutic agent (hereinafter, described primarily in the context of delivery of a medical device) to the selected location. In general, the patient may be a human patient. However, in other examples, the patient may be a non-human patient. The selected location may generally include any site within the patient where stimulation, sensing, drug delivery, or therapy is desired. In some examples, the selected locations may include tissue suitable for ablation, such as ablation using cold, heat, electrical energy, or radiation. In some examples, the selected location includes a cardiac tissue, a coronary vein, or tissue suitable for pacing, which is not dead, damaged, or otherwise not operating within general anatomical norms. In some examples, the medical device may include a medical electrical lead, such as a brady lead or a tachy lead; a site selective medical electrical lead, such as His bundle or septal pacing lead; an implantable medical device (IMD), such as implantable pacing devices; or a left bundle branch medical electrical lead.

In this disclosure, the example systems, devices, and techniques will be described with reference to delivering a medical electrical lead to a selected location in a heart. However, it will be understood that example systems, devices, and techniques of the present disclosure are not limited to delivering medical electrical leads to heart tissue. For example, example systems, devices, and techniques described herein may be used to deliver medical electrical leads to a coronary vein, to epicardial tissue, or other locations. Additionally, example systems, devices, and techniques described herein may be used to deliver medical electrical leads for neurostimulation therapy (e.g., spinal cord stimulation), deep brain stimulation, stimulation of one or more muscles, muscle groups or organs, and, generally, stimulation of tissue of a patient. Further, in some examples, the example systems, devices, and techniques described herein can be used to deliver medical devices for dispensing a drug or other beneficial agent from an implanted or external drug delivery device. Additionally, in some examples, the example system, devices, and techniques described herein can be used to deliver medical devices for ablating tissue using for example, cold, heat, electrical energy, or radiation. In short, the example systems, devices, and techniques described herein can find useful application in delivery of a wide variety of medical electrical leads or catheters for delivery of therapy to a patient or patient sensing.

To overcome the drawbacks of some medical imaging techniques, such as fluoroscopy, as discussed above, ultrasound may be used to guide a medical device (e.g., a medical balloon, a tube, or a delivery catheter) to a selected location within a patient. For example, echogenic particles may be incorporated into a medical balloon to provide echogenicity and/or radiopacity. The use of echogenic particles may cause the medical balloon to have a lower burst pressure than a medial balloon without having particulate additive. The consequences of a lower burst pressure may result in a weaker support structure and may shorten the lifespan and reduce the reliability of the medical balloon or preclude the use of the balloon from high pressure applications like angioplasty and stent deployment. The disclosure is directed to systems and techniques for facilitating delivery of an echogenic medical device to a selected location using ultrasound while providing a strong support structure that is resistant to bursting and tearing.

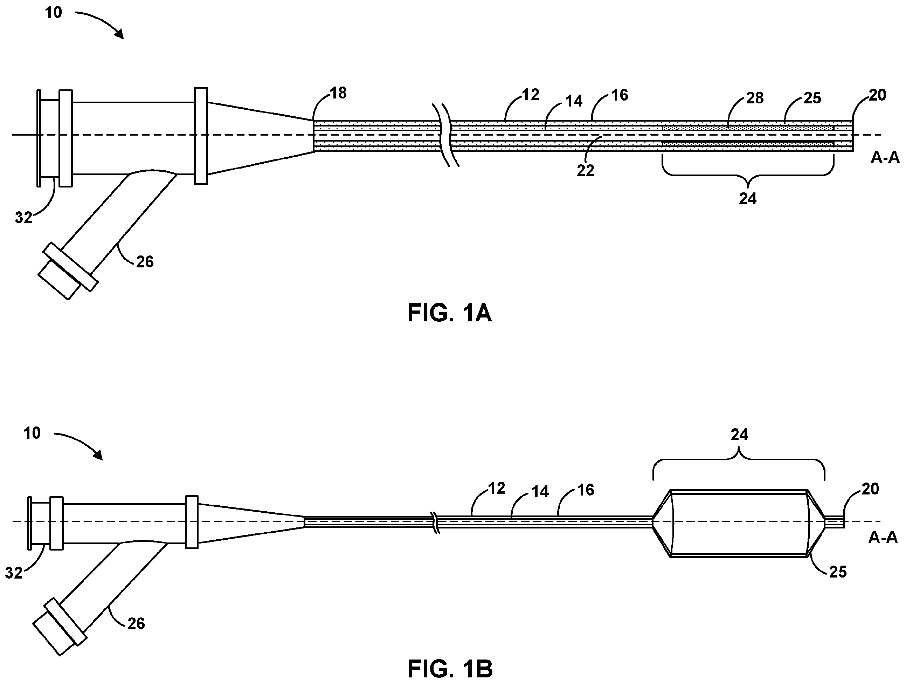

FIG. 1A is a schematic and conceptual side view of an example medical device 10 having an echogenic region 24. Medical device 10 includes multiple layers, such that echogenic region 24 of medical device 10 is a multilayer echogenic region that is resistant to bursting and tearing. As illustrated in FIG. 1, medical device 10 includes first layer 12 and second layer 14.

First layer 12 defines an elongated body 16 extending along a central longitudinal axis A-A from a proximal end 18 of elongated body 16 to a distal end 20 of elongated body 16. Second layer 14 is disposed radially adjacent to the first layer 12 and defines a lumen 22 extending longitudinally within elongate body 16. Lumen 22 may be configured to receive a medical electrical lead or IMD. For example, lumen 22 may be sized to pass a medical electrical lead through an entire length of lumen 22. In some examples, elongate body 16 may include a plurality of lumens, each lumen extending along and/or parallel to central longitudinal axis A-A. At least a portion of second layer 14 defines echogenic region 24 located closer to distal end 20 of elongated body 16 than proximal end 18 of elongated body 16.

The elongated body 16 may have any suitable dimensions, which may depend upon the medical procedure with which the medical device 10 is intended to be used. For example, the elongated body 16 can have any suitable length, such as, but not limited to, about 20 centimeters (cm) to about 150 cm, such as about 75 cm, about 90 cm, or about 135cm (e.g., exactly these lengths or approximately these lengths to the extent permitted by manufacturing tolerances).

In some examples, an outer diameter of the elongated body 16 may be about 2 French to about 12 French, such as about 3 French or about 6 French. The measurement term French, abbreviated Fr or F, is three times the diameter of a device as measured in millimeter (mm). Thus, a 6 French diameter is about 2 mm (e.g., about 1.8 mm), a 5 French diameter is about 1.67 mm, a 4 French diameter is about 1.33 mm, and a 3 French diameter is about 1 mm. The term "about" as used herein with dimensions may refer to the exact value of the such as when used to describe numerical values, "about" or "approximately" refers to a range within the numerical value resulting from manufacturing tolerances and/or within 1%, 5%, or 10% of the numerical value. For example, a length of about 10 mm refers to a length of 10 mm to the extent permitted by manufacturing tolerances, or a length of 10 mm +/- 0.1 mm, +/- 0.5 mm, or +/- 1 mm in various examples.

Echogenic region 24 may be located any suitable distance from distal end 20 of elongated body 16 to enable a clinician to determine the relative position of distal end 20. In some examples, echogenic region 24 may be within a range from about 0 mm (e.g., a distal end of echogenic region 24 may be flush or nearly flush within manufacturing tolerances relative to distal end 20 of elongated body 16) to about 2 mm proximal to distal end 20. In other examples, echogenic region 24 may be greater than 2 mm proximal to distal end 20. In some examples, echogenic region 24 may be located at other locations on medical device 10, such as, for example, adjacent preformed curves or articulating portions of medical device 10.

First layer 12 of medical device 10 may be formed from any suitable polymer. In some examples, first layer 12 may include one or more of acrylonitrile-butadiene styrene (ABS), polyamides, for example, nylons, polyamide 6 (PA 6), or polyamide 66 (PA 66), polycarbonates (PC), polyethylenes (for example, high density polyethylenes (HDPE) or low density polyethylenes (LDPE)), poly(methyl methacrylate) (PMMA), polyoxymethylene (POM), polypropylenes (PP), polystyrenes (PS), polybutylene terephthalate (PBT), styrene acrylonitrile resin (SAN), thermoplastic elastomers (TPE) (for example, polyether block amides (PEBAs)), polyphenylene sulfide (PPS), polyetheretherketones (PEEK), polyurethanes, polyesters, or blends, copolymers, or coextrusions thereof. In some examples, the TPEs (or PEBAs) may include materials sold under the PEBAX^{®} brand name (Arkema, Paris, France) or VESTAMID ^{®} (Evonik Industries, Essen, Germany).

Second layer 14 of medical device 10 can be made of a variety of materials, including the same materials as of the first layer 12. In some examples, first layer 12 and second layer 14 may be made of the same material and may have substantially the same density (e.g., the same but for manufacturing tolerances). In other examples, first layer 12 and second layer 14 may be made of different materials and may have different densities. For example, first layer 12 may be made of a first polymer and second layer 14 may be made of a second polymer. The first polymer may have a first density within a range from about 0.7 g/cm³ to about 5.5 g/cm³ and the second polymer may have a second density within a range from about 0.7 g/cm³ to about 5.5 g/cm³.

In addition, in some examples, first layer 12 and second layer 14 may have substantially the same radial thicknesses (e.g., the same but for manufacturing tolerances), where a radial thickness is measured in a direction orthogonal to central longitudinal axis A-A. In other examples, first layer 12 and second layer 14 have different radial thicknesses. For example, first layer 12 may have a first radial thickness within a range from about 0.001 mm to about 2 mm, and second layer 14 may have a second radial thickness within a range from about 0.001mm to about 2 mm. The radial thickness of each of first layer 12 and second layer 14 may depend upon the intended use of medical device 10.

Echogenic region 24 of second layer 14 may include embedded echogenic particles 28. In some examples, echogenic particles 28 may include an echogenic material or an echogenic metallic or ceramic material, such as one or more of tungsten, tantalum, platinum, gold, stainless steel, aluminum, titanium, aluminum oxide, magnesium oxide, silica glass, silicon dioxide, tungsten carbide, silicon carbide, titanium dioxide, titanium carbide, or titanium nitride. In some examples, echogenic particles 28 may have an average diameter within a range from about 0.1 micrometer (µm) to about 50 µm. Echogenic particles 28 embedded in echogenic region 24 are configured to enhance the acoustic impedance and diffuse scattering characteristics of echogenic region 24 and the average diameter of echogenic particles 28 may be selected to strike a balance between diffuse scattering and the device manufacturability. For example, the selected diameter of echogenic particles 28 may help to ensure a low particle sedimentation rate in the case where the second layer were formed by a coating process. Additionally, the selected diameter of echogenic particles 28 may help to enhance mechanical properties of medical device 10, such as enhancing burst pressure and tensile strength of medical device 10. Instead of or in addition to echogenic region 24 of second layer 14, first layer 12 may possess an echogenic region (not shown) that can optionally be similar in configuration, material composition, and/or location to any of the various disclosed embodiments of echogenic region 24.

In some examples, echogenic region 24 has a selected density within a range from about 1.0 gram per cubic centimeter (g/cm³) to about 16.0 g/cm³, such as from about 1.0 g/cm³ to about 4.0 g/cm³, 1.5 g/cm³ to about 3.0 g/cm³, 2.0 g/cm³ to about 3.5 g/cm³ The density of echogenic region 24 is selected so that the difference between the density of the echogenic region 24 and first layer 12 is within a range from about 0 cm³/g to about 15 g/cm³. In addition, echogenic region 24 also has a selected specific acoustic impedance within a range from about 2 mega Rayls (MRayl) to about 90 MRayl. The density of echogenic region 24 and the specific acoustic impedance of echogenic region 24 may be selected to strike a balance between a high specific acoustic impedance and a low particle sedimentation rate or density mismatch between the first and second layer.

Echogenic region 24 may be configured to diffusely scatter soundwaves, such as soundwaves having a frequency greater than about 20,000 Hertz (Hz), such as greater than about 1 mega Hertz (MHz), such as within a range from about 1 MHz to about 20 MHz.

In some examples, first layer 12 defines an outermost surface of medical device 10, while second layer 14 defines an innermost surface of medical device 10. However, in some examples, medical device 10 may include one or more additional layers. Additionally, or alternatively, one or more coatings or surface treatments may be applied to first layer 12 and/or second layer 14, such as, but not limited to, a lubricious coating, a lubricious surface treatment or a therapeutic agent. By having multiple layers, such as both first layer 12 and second layer 14, medical device 10 provides a strong support structure that is resistant to bursting and tearing.

In some examples, medical device 10 may include an expandable portion 25 controllable between a collapsed configuration and an expanded configuration. In the collapsed configuration, a diameter of echogenic region 24 may be sufficiently small to pass through the vasculature of the patient, such as equal to or less than about 28 Fr (9.333 mm), or less than about 9.5 mm. In the expanded configuration, a size and/or a shape of echogenic region 24 may be selected to improve visualization of echogenic region 24 using ultrasound. In some examples, a diameter of echogenic region 24 in an expanded configuration may be within a range from about 1 mm to about 30 mm, such as about 2 mm to about 15 mm. The diameter of echogenic region 24 may be selected to facilitate imaging of medical device 10 and/or facilitate maneuverability through the vasculature or the heart of the patent. In the collapsed configuration, expandable portion 25 may remain relatively adjacent to central longitudinal axis A-A, such as a diameter of expandable portion 25 may be substantially similar to a diameter of other portions of elongate body 16. In this way, expandable portion 25 may not affect the introduction of medical device 10 through the vasculature of the patient. In the expanded configuration, expandable portion 25 may be configured to anchor medical device 10 to a target site. In addition, expandable portion 25 may be configured to improve visualization in the heart or other portions of the body to provide a larger target to pick up on ultrasound. FIG. 1B is a schematic and conceptual side view of medical device 10 in an expanded configuration. In other examples, medical device 10 may not include expandable portion 25.

As illustrated in FIG. 1B, echogenic region 24 is located at expandable portion 25 of medical device 10. By including echogenic region 24 at expandable portion 25, medical device 10 may be configured to provide a larger ultrasound target (e.g., relative to a non-expandable portion of medical device 10). The larger ultrasound target may enable a clinician to more accurately visualize and, thereby, guide medical device 10 to a selected location within a patient.

Medical device 10 further includes a hub 26 connected to the proximal end 18 of elongated body 16 may allow elongated body 16 to be manipulated, advanced, or retracted, and may provide ports for communicating with lumens defined by elongated body 16. For example, hub 26 may include an inflation arm that may be connected to a source of inflating fluid to deliver inflating fluid to inflate a portion of elongated body 16, or deflate a portion of elongated body 16 by withdrawing the inflating fluid. In some examples, hub 26 may include an adapter 32 to receive a guidewire through a guidewire lumen in elongated body 16 (not shown). In some examples, elongated body 16 may include a catheter body, for example, a balloon catheter, and hub 26 may include a catheter hub. In some examples, instead of a guidewire catheter, medical device 10 may include a rapid-exchange balloon catheter system.

Elongated body 16 may be advanced to a target site, for example, through a body lumen such as a blood vessel of a patient. In some examples, distal end 20 of elongated body 16 may be introduced into the vasculature of the patient through an incision or opening, followed by a shaft of elongated body 16. Elongated body 16 may be advanced through the body lumen, for example, over a guidewire introduced through adapter 32 of hub 26. Elongated body 16 may be maintained in collapsed or partly expanded configuration while advancing elongated body 16 through the vasculature. When elongated body 16 is sufficiently advanced, for example, such that echogenic region 24 is adjacent the target site, inflating fluid may be delivered to inflate a portion of the elongated body 16 to expanded configuration at the target site.

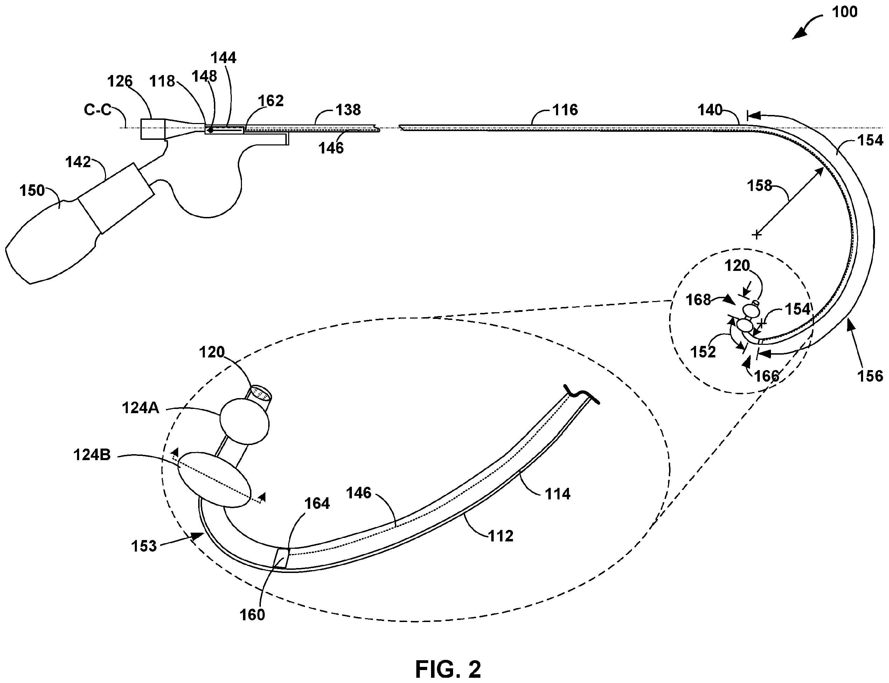

In some examples, a medical device may include more than one echogenic region. FIG. 2 is a conceptual diagram illustrating an example medical device 100 including a first layer 112 and a second layer 114, where second layer 114 defines echogenic regions 124A and 124B (collectively, "echogenic regions 124"). Medical device 100 may be the same as or substantially similar to medical device 10 discussed above in reference to FIGS. 1A and 1B, except for the differences described herein.

First layer 112 defines an elongate body 116 extending from a proximal end 118 to a distal end 120. Elongate body 116 may include any suitable length to reach the selected location of the heart from an access site, such as a femoral access site or a radial access site. Elongate body 116 may include proximal portion 138 near proximal end 118 and distal portion 140 near distal end 120. Elongate body 116 has a flexibility allowing deflection of distal portion 140 when elongate body 116 is maneuvered within the vasculature of a patient. Proximal portion 138 may be coupled to handle assembly 142 having a control member 144. Proximal portion 138 extends along longitudinal axis C-C. In some examples, proximal portion 138 may include a stabilizing sheath that surrounds proximal portion 138 and is configured to transfer force, such as torque, at handle assembly 142 to distal portion 140.

In some examples, handle assembly 142 may include a hub 126, adjustable handle 150, and/or control member 144. Hub 126 may be configured to provide access to a lumen of elongate body 116. For example, hub 126 may provide access to a lumen fluidly coupled to echogenic regions 124 that include inflatable balloons. In this way, a clinician may use a fluid to inflate and/or deflate echogenic regions 124, for example, via a syringe. In some examples, handle assembly 142 may include a flushing assembly configured to couple to a syringe to, for example, purge air from lumens of medical device 100. Adjustable handle assembly 142 may be configured to manipulate, e.g., rotate, the deflection of distal portion 140. Control member 144 may include one or more controls 148 that are coupled to one or more pull wires 146. One or more controls 148 may be manipulated to control a length of pull wire 146 extending through elongate body 116.

Echogenic regions 124 are located on distal portion 140 of elongate body 116. In some examples, echogenic regions 124 are configured to controllably expand from a collapsed configuration to an expanded configuration and diffusely scatter soundwaves. Echogenic region 124A is located near distal end 120. For example, echogenic region 124A may be located proximal distal end 120 a distance within a range from about 0 mm to about 2 mm. Echogenic region 124B is proximal to echogenic region 124A. For example, echogenic region 124B may be space apart from echogenic region 124A a distance within a range from about 1 mm to about 30 mm, such as about 5 mm to about 25 mm or about 10 mm to about 20 mm. In some examples, echogenic regions 124A and 124B may be directly adjacent. In examples in which echogenic regions 124A and 124B are directly adjacent, the volume of echogenic regions 124A and 124B that expands (e.g., a maximum diameter of the expanded configuration) may be separated a distance within a range from about 1 mm to about 30 mm, such as about 5 mm to about 25 mm or about 10 mm to about 20 mm.

In some examples, echogenic regions 124, spaced apart by a selected distance, may enable a clinician to determine an orientation and/or a trajectory of medical device 100 relative to surrounding soft tissue. For example, by visualizing both echogenic regions 124 in the plane of an ultrasound, a clinician may determine that at least distal portion 140 including the echogenic regions 124 is oriented in the plane. This enables determining that an orientation and/or trajectory of at least distal portion 140 is in the plane of the surrounding anatomy of the patient indicated by the ultrasound. Determining the orientation and/or trajectory of distal portion 140 may facilitate traversing valves of the heart or tunneling between chambers of the heart, such as from the right atrium to the left ventricle or from the right ventricle to the left ventricle. Compared to other medical imaging techniques having reduced soft tissue visibility, such as fluoroscopy, the use of echogenic regions to determine an orientation and/or a trajectory of medical device 100 relative to a surrounding anatomy using ultrasound may improve clinician confidence and speed with respect to navigating the vasculature of a patient.

The size and shape of the first and second echogenic regions may be similar or dissimilar. For example, as illustrated in FIG. 2, echogenic region 124A may be smaller and more spherical compared to echogenic region 124B. This dissimilarity in size and/or shape may enable a clinician to distinguish between echogenic regions 124A and 124B. Distinguishing between echogenic regions 124A and 124B may enable determining an orientation and/or a trajectory of distal portion 140. In some examples, the distance between echogenic regions 124 may be based on the size and/or shape of echogenic regions 124. For example, an ultrasound image showing the total diameter of echogenic region 124A may indicate that echogenic region 124A is centered in the plane of the surrounding anatomy indicated by the ultrasound image. When the ultrasound image shows a portion of echogenic region 124B less than the total diameter of echogenic region 124B, the clinician may determine that an orientation and/or trajectory of medical device 100 is either above or below the plane of the ultrasound image. In some examples, smaller echogenic regions may enhance resolution of the alignment with the ultrasound plane compared to larger echogenic regions. In some examples, larger echogenic regions may be easier to track compared to smaller echogenic regions. In this way, the size and spacing of echogenic regions 124 may be selected to improve visualization of medical device 100 (e.g., distal portion 140) using ultrasound which may improve determination of a trajectory and an orientation of distal portion 140 relative to surrounding tissue compared to other medical imaging techniques, such as fluoroscopy.

Although described as including two echogenic regions 124, in some examples, medical device 100 may include three or more echogenic regions located on distal portion 140 of medical device 100 to enable a clinician to determine a position, orientation, and/or trajectory of selected portions of medical device 100, including, for example, distal end 120, preformed curves 152, or articulating segment 154. For example, distal portion 140 may include an articulating segment 154 and a preformed curve segment 152 distal articulating segment 154. In this way, a shape of distal portion 140 may be controllable. For example, pull wire 146, e.g., by actuation of control member 144, may be configured to controllably bend articulating segment 154 in a first curve 156 in a first geometric plane. In some examples, the amount of actuation of control member 144 may control the degree of curvature of articulating segment 154. For example, a degree of curvature of articulating segment 154 may be controlled in a range between about 0 degrees to about 240 degree, such as between about 45 degrees and about 180 degrees or between about 85 degrees and about 100 degrees. In some examples, a length of articulating segment 154 defining first curve 156 may be within a range from about 5 cm to about 20 cm, such as from about 12 cm to about 15 cm. In some examples, a radius 158 of first curve 156, when articulated, may be within a range between about 5 mm and about 60 mm, such as between about 10 mm and about 30 mm or between about 15 mm and about 20 mm. By controlling the degree of curvature, articulating segment 154 may enable first curve 156 to be adjusted to accommodate a variation in the position of a selected location or a difference in size of a dilated heart compared to an average sized heart.

Pull wire 146 may enable control of the degree of curvature of articulating segment 154 from handle assembly 142. For example, a proximal end 162 of pull wire 146 may be coupled to control member 144. Pull wire 146 may extend from control member 144 to a distal end 164 of pull wire 146 anchored to elongate body 116 distal to articulating segment 154. For example, distal end 164 may be anchored to elongate body 116 using a pull band 160. Pull band 160 may include any suitable structure configured to anchor a distal end 164 of pull wire 146 to elongate body 116 distal articulating segment 154. In some examples, pull band 160 may include a radiopaque marker, gold, platinum iridium, other noble metals or alloys thereof, stainless steel, other materials configured to withstand deflection force from actuating pull wire 146 which may include sputtered noble metals, or combinations thereof. In some examples, pull wire 146 includes a single pull wire. In other example, pull wire 146 may include a plurality of pull wires. In examples in which pull wire 146 include a plurality of pull wires, each pull wire of the plurality of pull wires may be configured to control a deflection of distal portion 140 in one or more directions. Pull wire 146 may include any suitable material and construction. In some examples, pull wire 146 may have a diameter of approximately 0.009 inch (0.23 mm) and may be formed from medical grade stainless steel. In some examples, pull wire 146 may include a coating, e.g., a fluoropolymer, such as polytetrafluoroethylene (PTFE). By anchoring distal end 164 of pull wire 146, actuation of control member 144 in a proximal direction, e.g., to shorten a length of pull wire 146 extending through elongate body, may result in a controllable bending of articulating segment 154 in a geometric plane. Actuation of control member 144 in a distal direction, e.g., to lengthen a length of pull wire 146, may result in a controllable return of articulating segment 154 to a resting state, e.g., unbent or less bent configuration.

In some examples, a shape of distal portion 140 may include a preformed curve. For example, preformed curve segment 152 defines a second curve 153 in a second geometric plane. The second geometric plane may be similar or different from the first geometric plane. For example, first geometric plane and second geometric plane may be offset by an offset angle. In some examples, the offset angle, e.g., the angle of first geometric plane relative to second geometric plane, may be within a range from about 10 degrees to about 80 degrees, such as about 30 degrees to about 60 degrees or about 40 degree to about 50 degrees.

In some examples, preformed curve segment 152 may be sufficiently flexible to deform into a substantially straight configuration when passed through the vasculature of a patient. Preformed curve segment 152 may be sufficiently resilient to regain the preformed shape of second curve 153 when positioned in the heart of the patient. In some examples, second curve 153 of preformed curve segment 152 may be formed by, for example, heat setting. In some examples, a degree of curvature of preformed curve segment 152 may be in a range between about 10 degrees to about 180 degree, such as between about 30 degrees and about 140 degrees. In some examples, a length of preformed curve segment 152 defining second curve 153 is within a range between about 6 mm and about 10 cm, such as between about 1 cm and about 5 cm or between about 1 cm and about 2 cm. In some examples, a radius 155 of second curve 153 is within a range between about 1 mm and about 20 mm, such as between about 2 mm and about 10 mm. The degree of curvature of preformed curve segment 152 may enable the distal end 120 to be oriented substantially normal to tissue at the selected location.

In some examples, distal portion 140 may include one or more substantially straight portions. For example, elongate body 116 may include substantially straight portion 166 distal articulating segment 154 and proximal preformed curve segment 152, and/or substantially straight portion 168 distal to preformed curve segment 152 and including distal end 120. In some examples, a length of substantially straight portion 166 and/or 168 may be in a range between about 1 mm and about 15 mm, such as between about 0.5 mm and about 9 mm. In some examples, echogenic regions 124 may be located on one or more of substantially straight portions 166 or 168.

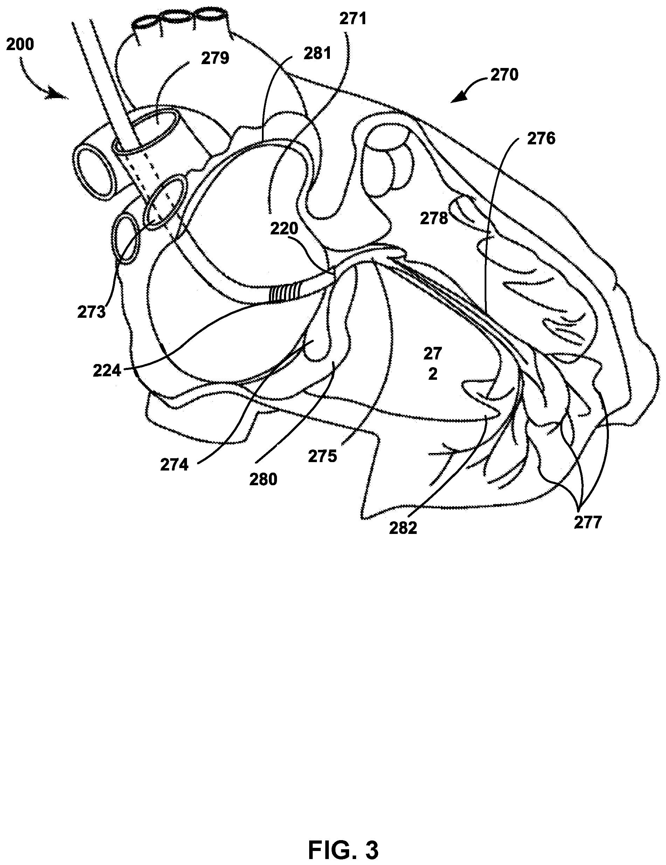

FIG. 3 is a conceptual diagram illustrating distal portion 240 of an example medical device 200 positioned in the right side of a heart 270. Medical device 200 may be the same as or substantially similar to medical device 10 or medical device 100 discussed above in reference to FIGS. 1-2.

Medical device 200 includes an echogenic region 224 located on distal portion 240 (e.g., near distal end 220) of medical device 200. Echogenic region 224 is configured to facilitate guiding medical device 200 to a selected location within heart 270 using ultrasound.

As illustrated in FIG. 3, heart 270 has an anterior-lateral wall peeled back to present a portion of the intrinsic conduction system of heart 270 and chambers of a right atrium (RA) 271 and a right ventricle (RV) 272. Pertinent elements of the intrinsic conduction system of heart 270 may include a sinoatrial (SA) node 273, an atrioventricular (AV) node 274, a His bundle 275, a right bundle branch 276, and Purkinje fibers 277. SA node 273 is shown near the superior vena cava (SVC) 279 in the RA 271. An electrical impulse starting at the SA node 273 travels rapidly through tissue of RA 271 and tissue of a left atrium (not shown) to AV node 274. At AV node 274, the impulse slows to create a delay before passing on through His bundle 275, which branches, in an interventricular septum 278, into a right bundle branch 276 and a left bundle branch (not shown) and then, near RV apex 16, into Purkinje fibers 277. Flow of the electrical impulse creates an orderly sequence of atrial and ventricular contraction and relaxation to efficiently pump blood through heart 270.

Due to disease, injury, or natural defects, the intrinsic conduction system of heart 270 may no longer operate within general anatomical norms. In some examples, a cardiac pacemaker system can be implanted into a patient such that electrodes carried by an implantable medical electrical lead or a leadless implantable medical device (IMD) may be placed in an atrial appendage 281. The electrodes stimulate RA 271 downstream of SA node 273 and the stimulating pulse travels on to AV node 274, His bundle 275, and Purkinje fibers 277 to restore physiological contraction of the heart. However, if a patient has a defective AV node 274 pacing in atrial appendage 281 will not be effective, since the pacing site is upstream of AV node 274, e.g., atrioventricular block. For these or other reasons, a patient may have a cardiac pacemaker system implanted such that medical electrical leads are positioned at selected locations in RV apex 16, the His bundle 275 (as illustrated in FIG. 1A), the ventricular septum, or suitable locations in the left atrium or left ventricle. Navigating medical device 200 to deliver an electrical lead or a leadless implantable medical device (IMD) to a selected location within a patient requires medical imaging to visualize the location of medical device 200 relative to anatomy of heart 270. In some examples, medical device 200 may be inserted into heart 270 using a transvenous approach through the SVC 279 into the RA 271. In some examples, medical device 200 may be directed through tricuspid valve 280 to RA 272. In some examples, medical device 200 may be tunneled from the right atrium, either through the interatrial septum and interventricular septum to the left ventricle or through the right ventricle and interventricular septum to the left ventricle. To overcome the drawbacks of some medical imaging techniques, such as fluoroscopy, as discussed above, ultrasound may be used to guide medical device 200 to a selected location within heart 270.

Positioning medical device 200 using ultrasound may be difficult due to the nature of ultrasound imaging. For example, the field of view of an ultrasound image may include a two-dimensional (2D) plane having a thickness within a range from about 2 millimeters (mm) to about 6 mm. Because the 2D plane of the ultrasound image is thin, e.g., relative to the volume of the heart 270 and/or the relative motion of medical device 200 as it is advanced to a selected location, it may be difficult for a clinician to maintain medical device 200 in the field of view, such as, for example, during motion caused by heart beats. Additionally, the resolution of ultrasound imaging may make it difficult to distinguish features smaller than about 1 mm to about 2 mm in diameter. Also, to visualize a structure using ultrasound, the structure must reflect (e.g., scatter) at least a portion of an emitted ultrasonic soundwave back to an ultrasound transducer. As used herein, visualizing a structure using ultrasound means to detect the structure by receiving at an ultrasound transducer a signal indicative of a reflection of ultrasonic sound waves emitted from the ultrasound transducer and processing the signal to generate an image indicative of the structure. In some examples, smooth surfaces of medical devices, such as medical device 200, may produce geometric scattering of an ultrasonic soundwave rather than diffuse scattering of the ultrasonic soundwave. Geometric scattering may reduce visualization of the medical device at angles off perpendicular. For these reasons, it may be difficult for a clinician to orient an ultrasound transducer to visualize medical device 200, to determine which portion of medical device 200 (or the medical device to be delivered) is within the field of view of the ultrasound, or both.

Echogenic region 224 may facilitate guiding medical device 200 to a selected location within heart 270 using ultrasound by increasing the size and diffuse reflection of at least a portion of medical device 200. Medical device 200 may comprise a flexible, biocompatible material such as, for example, silicone or polyurethane. In some examples, medical device 200 may include a preformed curve. For example, upon advancing into RA 271, medical device 200 may begin to regain its preformed curve. In some examples, medical device 200 is a steerable catheter. In some examples, medical device 200 is a guidable catheter and includes a lumen for receiving a guide wire to assist with advancing the medical device 200 at least a portion of a distance to a selected location within heart 270. In some examples, medical device 200 includes features that allow it to effectively transfer force applied to a proximal end, e.g., via a handle assembly (not shown), of medical device 200 into motion of a distal end 220 of medical device 200. For example, distal portion 240 may include multiple curves proximate distal end 220 to facilitate guiding distal end 220 to a selected location. In some examples, the multiple curves may be formed by an articulating segment adjustable by, for example, a pull wire that can be manipulated by a control member at the handle assembly, a preformed curve segment, or other features configured to shape a length of distal portion 240.

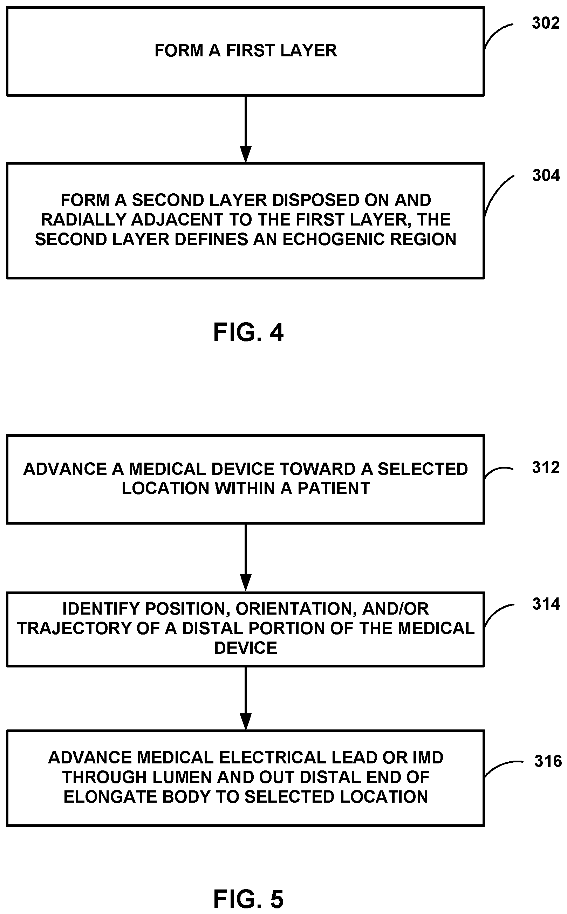

The medical devices described herein may be assembled by any suitable technique. FIG. 4 is a flow diagram illustrating an example method for assembling an example medical device having an echogenic region. The medical device may be the same as or substantially similar to medical devices 10, 100 and/or 200 discussed above with respect to FIGS. 1-3. Although FIG. 4 is described with respect to medical device 10, in other examples, the method of FIG. 4 may be used to assemble other medical devices having echogenic regions.

The technique illustrated in FIG. 4 includes forming first layer 12 (302). As discussed above, first layer 12 may define an elongated body 16 extending along longitudinal axis A-A from proximal end 18 of elongated body 16 to distal end 20 of elongated body 16 and may define lumen 22 extending longitudinally within elongate body 16. First layer 12 of medical device 10 may be formed from any suitable polymer. Lumen 22 may be configured to receive a medical electrical lead that includes at least one electrode and/or an IMD.

The technique illustrated in FIG. 4 also includes forming second layer 14 (304). As discussed above, second layer 14 may be disposed on and radially adjacent to the first layer 12. Second layer 14 may be fixed to first layer 12 by, for example, an adhesive, thermo-welding, or ultrasonic welding. In some examples, second layer 14 may be coated on first layer 12, for example, by dip coating or spray coating. Conversely, first layer 12 may be coated upon second layer 14.

A portion of second layer 14 may define echogenic region 24. Echogenic region 24 of second layer 14 may have embedded echogenic metallic or ceramic material, such as echogenic particles 28. In some examples, echogenic particles 28 may include one or more of tungsten, tantalum, platinum, gold, stainless steel, aluminum, titanium, aluminum oxide, magnesium oxide, silica glass, silicon dioxide, tungsten carbide, silicon carbide, titanium dioxide, titanium carbide, or titanium nitride. For example, second layer 14 may be a silicon adhesive layer containing echogenic particles 28 and may be dip coated upon first layer 12. In one example, echogenic particles 28 may include lightweight low density particles (e.g., aluminum particles) to help to prolong the particles suspension time.

First layer 12 may be formed by any suitable technique, such as, for example, heating and inflating a first polymer within a mold. Second layer 14 may be formed by inserting a second polymer into the formed first layer 12 and heating and inflating the second polymer in a similar way. As above, this process may be repeated to form additional layers, such as a third layer.

In some examples first layer 12 and second layer 14 may be sequentially extruded prior to inflation within a mold. For example, first layer 12 may be formed of a polymeric material and may be extruded prior to inflation within a balloon mold. The balloon mold may be heated to an elevated temperature to soften the polymeric material of first layer 12 for the molding process. The softened polymeric material of first layer 12 may then be inflated to expand the softened polymetric material within the cavity of the balloon mold such that the polymetric material conforms to the shape of the cavity to form the desired shape. Second layer 14 may include the same polymeric material as first layer 12 and may be sequentially extruded prior to inflation within the balloon mold. The polymeric material of second layer 14 may then be heated and inflated to bond to first layer 12. In some examples, the initial sequential extrusion can be conducted to form three or more layers prior to inflation within the balloon mold.

In some examples, the second polymer is placed over the first polymer prior to shaping both polymers to form first layer 12 and second layer 14. The first and second polymers may be sequentially extruded tubes in such examples. The first and second polymers may be laminated together before, during, or after the shaping.

In some examples, first layer 12 and second layer 14 may be coextruded prior to inflation within a mold. For example, first layer 12 may be formed of a first polymeric material and second layer 14 may be formed of a second polymeric material, and first layer 12 and second layer 14 may be coextruded prior to inflation within a balloon mold. The balloon mold may be heated, and first layer 12 and second layer 14 may be inflated to form multilayer echogenic region 24. Coextruding first layer 12 and second layer 14 may help to bond first layer 12 formed of the first polymeric material with second layer 14 formed of the second polymeric marital. In some examples, the initial coextrusion can be conducted to form three or more layers prior to inflation within the balloon mold.

In some examples, echogenic region 24 of medical device 10 may further include an innermost layer configured to be bonded to a shaft (e.g., a catheter shaft) of elongated body 16 of medical device 10. The innermost layer may be made of a material that is compatible with bonding the innermost layer with to the shaft of elongated body 16. In some examples, the innermost layer may be made of a material different from the materials of first layer 12 and second layer 14.

In some examples, the technique may include applying an echogenic coating to first layer 12 and/or second layer 14. For example, first layer 12 and/or second layer 14 may be dip-coated in an echogenic coating or an echogenic coating may be injected into first layer 12 and/or second layer 14 after forming first layer 12 and/or second layer 14. Echogenic medical device 10 with first layer 12 and second layer 14 is configured to be deployed to a selected location using ultrasound while having a strong support structure that is resistant to bursting and tearing. The medical devices described herein may be used to deliver a medical electrical lead or IMD using any suitable technique. FIG. 5 is a flow diagram illustrating an example method of delivering a medical electrical lead to a selected location using an example medical device having an echogenic region. The medical device may be the same as or substantially similar to medical devices 10, 100 and/or 200 discussed above with respect to FIGS. 1-3. Although FIG. 5 is described with respect to medical device 100, in other examples, the method of FIG. 5 may be used to assemble other medical devices having echogenic regions.

The technique illustrated in FIG. 5 includes advancing medical device 100 toward a selected location within a patient (312). In some examples, after advancing medical device 100 toward the selected location, the technique may include actuating control member 144, e.g., via one or more controls 148, to cause pull wire 146 to controllably bend articulating segment 154 into first curve 156.

In some examples, the technique may include expanding echogenic region 124 from a collapsed configuration to an expanded configuration. In some examples, expanding echogenic region 124 may include injecting a fluid, such as saline, into echogenic region 124 via one or more lumens of medical device 100. In some examples, expanding echogenic region 124 may include actuating control member 144, e.g., via one or more controls 148, to cause pull wire 146 to expand echogenic region 124.

The technique illustrated in FIG. 5 also includes identifying at least one of a position, an orientation, or a trajectory of the distal portion of the catheter relative to the selected location based on a soundwave reflected by echogenic region 124 (314). In some examples, identifying may include imaging, by an ultrasound imaging device, the at least one echogenic region 124. For example, imaging may include capturing a plurality of images of the at least one echogenic region 124 and surrounding anatomy. Each image of the plurality of images may include different angles of an ultrasound transceiver relative to the medical device 100 and/or the surrounding anatomy. In some examples, identifying may include determining, based on the ultrasound image, a position, an orientation, and/or a trajectory of distal portion 140 (e.g., distal end 120) relative to the surrounding anatomy. For example, determining a position, an orientation, and/or a trajectory of distal portion 140 may include comparing a size and/or a shape of echogenic region 124 to a known size and/or shape of one or more cross sectional geometries of echogenic region 124. In some examples, determining a position, an orientation, and/or a trajectory of distal portion 140 may include comparing a size and/or a shape of a first echogenic region 124A to a size and/or a shape of a second echogenic region 124B. In some examples, after determining the position, the orientation, and/or the trajectory of distal portion 140, the technique may include further advancing, repositioning, or reorienting distal portion 140, and repeating imaging the at least one echogenic region 124.

The technique illustrated in FIG. 6 also includes, after identifying the selected location, advancing a medical electrical lead or an IMD through lumen and out distal end 120 of elongate body 116 to the selected location (316). For example, the technique may include advancing the medical electrical lead out of distal end 120 of medical device 100 and controlling a fixation member, e.g., via a lead body of the medical electrical lead controllable at or near a handle assembly of medical device 100, to screw the fixation member into the tissue at the selected location.

Various examples of the disclosure have been described. Any combination of the described systems, operations, or functions is contemplated. These and other examples are within the scope of the following claims.

## Claims

1. A medical device, comprising:
a first layer (12) defining an elongated body (16) extending along a longitudinal axis (A-A) from a proximal end (18) to a distal end (20) and defining a lumen (22) extending longitudinally within the elongate body; and
a second layer (14) disposed on and radially adjacent to the first layer,
wherein at least a portion of the second layer defines an echogenic region (24) comprising an echogenic metallic or ceramic material (28) dispersed in the second layer,
wherein the first layer comprises a first polymer, the second layer comprises a second polymer, and the echogenic metallic or ceramic material is dispersed in the second polymer, **characterised in that** the echogenic region is configured to diffusely scatter a soundwave and **in that**
a difference between a density of the echogenic region and a density of the first polymer is within a range from about 0 g/cm³ to about 15 g/cm³.

2. The medical device of claim 1, wherein the medical device comprises at least one of a medical balloon, a medical tube, or a catheter.

3. The medical device of claim 1 or 2, wherein the echogenic metallic or ceramic material comprises at least one of tungsten, tantalum, platinum, gold, stainless steel, aluminum, titanium, aluminum oxide, magnesium oxide, silica glass, silicon dioxide, tungsten carbide, silicon carbide, titanium dioxide, titanium carbide, or titanium nitride.

4. The medical device of any one of claims 1 to 3, wherein a density of the first polymer is within a range from about 0.7 g/cm³ to about 5.5 g/cm³.

5. The medical device of any one of claims 1 to 4, wherein a density of the second polymer is within a range from about 0.7 g/cm³ to about 5.5 g/cm³.

6. The medical device of any of claims 1-5, further comprising a third layer radially adjacent to the second layer, wherein the third layer comprises a third polymer.

7. The medical device of any of claims 1-6, wherein the echogenic metallic or ceramic material has a density within a range from about 1 g/cm³ to about 20 g/cm³.; and/or wherein the echogenic metallic or ceramic material has a specific acoustic impedance within a range from about 10 MRayl to about 110 MRayl.

8. The medical device of any of claims 1-7, wherein a radial thickness of the first layer is within a range from about 0.001 mm to about 2 mm; and/or wherein a radial thickness of the second layer is within a range from about 0.001 mm to about 2 mm.

9. The medical device of any of claims 1-8, wherein the echogenic material comprises a plurality of particles, wherein an average diameter of the plurality of particle is within range from about 0.1 µm to about 50 µm.

10. The medical device of any of claims 1-9, wherein the soundwave that the echogenic region is configured to diffusely scatter comprises an ultrasonic soundwave having a frequency within a range from about 1 MHz to about 20 MHz.

11. The medical device of any of claims 1-10, wherein the first layer defines an outermost surface of the medical device; and/or wherein the second layer defines an innermost surface the medical device; and/or wherein the second layer is disposed on an inner surface of the first layer; and/or wherein the echogenic region is expandable together with an adjacent portion of the first layer.

12. A kit comprising:
a first medical device according to any of claims 1-11; and
a second medical device sized for delivery out of the distal end of the elongate body and configured for at least one of therapy delivery or sensing.

13. The kit of claim 12, wherein the first medical device comprises at least one of a medical balloon, a medical tube, or a catheter.

## Patentansprüche

1. Medizinische Vorrichtung, umfassend:
eine erste Schicht (12), die einen länglichen Körper (16) definiert, der sich entlang einer Längsachse (A-A) von einem proximalen Ende (18) zu einem distalen Ende (20) erstreckt, und ein Lumen (22) definiert, das sich in Längsrichtung innerhalb des länglichen Körpers erstreckt; und
eine zweite Schicht (14), die auf der ersten Schicht angeordnet ist und radial an diese angrenzt,
wobei mindestens ein Abschnitt der zweiten Schicht eine echogene Region (24) definiert, umfassend ein echogenes metallisches oder keramisches Material (28), das in der zweiten Schicht dispergiert ist,
wobei die erste Schicht ein erstes Polymer umfasst, die zweite Schicht ein zweites Polymer umfasst und das echogene metallische oder keramische Material in dem zweiten Polymer dispergiert ist, **dadurch gekennzeichnet, dass** die echogene Region konfiguriert ist, um eine Schallwelle diffus zu streuen, und **dass**
eine Differenz zwischen einer Dichte der echogenen Region und einer Dichte des ersten Polymers innerhalb eines Bereichs von etwa 0 g/cm³ bis etwa 15 g/cm³ liegt.

2. Medizinische Vorrichtung nach Anspruch 1, wobei die medizinische Vorrichtung mindestens eines von einem medizinischen Ballon, einem medizinischen Schlauch oder einem Katheter umfasst.

3. Medizinische Vorrichtung nach Anspruch 1 oder 2, wobei das echogene metallische oder keramische Material mindestens eines von Wolfram, Tantal, Platin, Gold, Edelstahl, Aluminium, Titan, Aluminiumoxid, Magnesiumoxid, Quarzglas, Siliciumdioxid, Wolframcarbid, Siliciumcarbid, Titandioxid, Titancarbid oder Titannitrid umfasst.

4. Medizinische Vorrichtung nach einem der Ansprüche 1 bis 3, wobei eine Dichte des ersten Polymers innerhalb eines Bereichs von etwa 0,7 g/cm³ bis etwa 5,5 g/cm³ liegt.

5. Medizinische Vorrichtung nach einem der Ansprüche 1 bis 4, wobei eine Dichte des zweiten Polymers innerhalb eines Bereichs von etwa 0,7 g/cm³ bis etwa 5,5 g/cm³ liegt.

6. Medizinische Vorrichtung nach einem der Ansprüche 1 bis 5, ferner umfassend eine dritte Schicht radial angrenzend an die zweite Schicht, wobei die dritte Schicht ein drittes Polymer umfasst.

7. Medizinische Vorrichtung nach einem der Ansprüche 1 bis 6, wobei das echogene metallische oder keramische Material eine Dichte innerhalb eines Bereichs von etwa 1 g/cm³ bis etwa 20 g/cm³ aufweist.; und/oder wobei das echogene metallische oder keramische Material eine spezifische akustische Impedanz innerhalb eines Bereichs von etwa 10 MRayl bis etwa 110 MRayl aufweist.

8. Medizinische Vorrichtung nach einem der Ansprüche 1 bis 7, wobei eine radiale Dicke der ersten Schicht innerhalb eines Bereichs von etwa 0,001 mm bis etwa 2 mm liegt; und/oder wobei eine radiale Dicke der zweiten Schicht innerhalb eines Bereichs von etwa 0,001 mm bis etwa 2 mm liegt.

9. Medizinische Vorrichtung nach einem der Ansprüche 1 bis 8, wobei das echogene Material eine Vielzahl von Partikeln umfasst, wobei ein durchschnittlicher Durchmesser der Vielzahl von Partikeln innerhalb eines Bereichs von etwa 0,1 µm bis etwa 50 µm liegt.

10. Medizinische Vorrichtung nach einem der Ansprüche 1 bis 9, wobei die Schallwelle, für deren diffuse Streuung die echogene Region konfiguriert ist, eine Ultraschallwelle umfasst, die eine Frequenz innerhalb eines Bereichs von etwa 1 MHz bis etwa 20 MHz aufweist.

11. Medizinische Vorrichtung nach einem der Ansprüche 1 bis 10, wobei die erste Schicht eine äußerste Oberfläche der medizinischen Vorrichtung definiert; und/oder wobei die zweite Schicht eine innerste Oberfläche der medizinischen Vorrichtung definiert; und/oder wobei die zweite Schicht auf einer inneren Oberfläche der ersten Schicht angeordnet ist; und/oder wobei die echogene Region zusammen mit einem angrenzenden Abschnitt der ersten Schicht expandierbar ist.

12. Kit, umfassend:
eine erste medizinische Vorrichtung nach einem der Ansprüche 1 bis 11; und
eine zweite medizinische Vorrichtung, die für eine Abgabe aus dem distalen Ende des länglichen Körpers bemessen und für mindestens eines von Therapieabgabe oder Erfassung konfiguriert ist.

13. Kit nach Anspruch 12, wobei die erste medizinische Vorrichtung mindestens eines von einem medizinischen Ballon, einem medizinischen Schlauch oder einem Katheter umfasst.

## Revendications

1. Dispositif médical, comprenant :
une première couche (12) définissant un corps allongé (16) s'étendant le long d'un axe longitudinal (A-A) d'une extrémité proximale (18) à une extrémité distale (20) et définissant une lumière (22) s'étendant longitudinalement à l'intérieur du corps allongé ; et
une deuxième couche (14) disposée sur la première couche et adjacente radialement à celle-ci,
dans lequel au moins une partie de la deuxième couche définit une région échogène (24) comprenant un matériau métallique ou céramique échogène (28) dispersé dans la deuxième couche,
dans lequel la première couche comprend un premier polymère, la deuxième couche comprend un deuxième polymère, et le matériau métallique ou céramique échogène est dispersé dans le deuxième polymère, **caractérisé en ce que** la région échogène est conçue pour diffuser de manière diffuse une onde sonore et **en ce que**
une différence entre une densité de la région échogène et une densité du premier polymère se situe dans une plage d'environ 0 g/cm³ à environ 15 g/cm³.

2. Dispositif médical selon la revendication 1, dans lequel le dispositif médical comprend au moins l'un parmi ballonnet médical, tube médical, ou cathéter.

3. Dispositif médical selon la revendication 1 ou 2, dans lequel le matériau métallique ou céramique échogène comprend au moins l'un parmi tungstène, tantale, platine, or, acier inoxydable, aluminium, titane, oxyde d'aluminium, oxyde de magnésium, verre de silice, dioxyde de silicium, carbure de tungstène, carbure de silicium, dioxyde de titane, carbure de titane ou nitrure de titane.

4. Dispositif médical selon l'une quelconque des revendications 1 à 3, dans lequel une densité du premier polymère se situe dans une plage d'environ 0,7 g/cm³ à environ 5,5 g/cm³.

5. Dispositif médical selon l'une quelconque des revendications 1 à 4, dans lequel une densité du deuxième polymère se situe dans une plage d'environ 0,7 g/cm³ à environ 5,5 g/cm³.

6. Dispositif médical selon l'une quelconque des revendications 1-5, comprenant en outre une troisième couche radialement adjacente à la deuxième couche, dans lequel la troisième couche comprend un troisième polymère.

7. Dispositif médical selon l'une quelconque des revendications 1-6, dans lequel le matériau métallique ou céramique échogène a une densité dans une plage d'environ 1 g/cm³ à environ 20 g/cm³ ; et/ou dans lequel le matériau métallique ou céramique échogène a une impédance acoustique spécifique dans une plage d'environ 10 MRayl à environ 110 MRayl.

8. Dispositif médical selon l'une quelconque des revendications 1-7, dans lequel une épaisseur radiale de la première couche se situe dans une plage d'environ 0,001 mm à environ 2 mm ; et/ou dans lequel une épaisseur radiale de la deuxième couche se situe dans une plage d'environ 0,001 mm à environ 2 mm.

9. Dispositif médical selon l'une quelconque des revendications 1-8, dans lequel le matériau échogène comprend une pluralité de particules, dans lequel un diamètre moyen de la pluralité de particules se situe dans une plage d'environ 0,1 µm à environ 50 µm.

10. Dispositif médical selon l'une quelconque des revendications 1-9, dans lequel l'onde sonore que la région échogène est conçue pour diffuser de manière diffuse comprend une onde sonore ultrasonore ayant une fréquence dans une plage d'environ 1 MHz à environ 20 MHz.

11. Dispositif médical selon l'une quelconque des revendications 1-10, dans lequel la première couche définit une surface la plus extérieure du dispositif médical ; et/ou dans lequel la deuxième couche définit une surface la plus intérieure du dispositif médical ; et/ou dans lequel la deuxième couche est disposée sur une surface interne de la première couche ; et/ou dans lequel la région échogène est expansible conjointement avec une partie adjacente de la première couche.

12. Kit comprenant :
un premier dispositif médical selon l'une quelconque des revendications 1-11 ; et
un second dispositif médical dimensionné pour une administration à partir de l'extrémité distale du corps allongé et conçu pour au moins l'une parmi une administration de thérapie ou une détection.

13. Kit selon la revendication 12, dans lequel le premier dispositif médical comprend au moins l'un parmi ballonnet médical, tube médical, ou cathéter.
